# EUROPÄISCHE PATENTANMELDUNG

(11) **EP 3 388 414 A1**
(43) Veröffentlichungstag der Anmeldung: **17.10.2018**
(21) Anmeldenummer: 17165994.9
(22) Anmeldetag: 11.04.2017
(51) Int. Cl.: C07C 45/50, C07C 47/33, C07C 47/43

(54) **VERFAHREN ZUR HYDROFORMYLIERUNG VON CYCLOOCTADIEN UNTER EINSATZ VON 4-([1,1':3',1''-TERPHENYL]-2'-YLOXY)-S-DINAPHTHO[2,1-D:1',2'-F][1,3,2]DIOXAPHOSPHEPIN**

(71) Anmelder: Evonik Degussa GmbH, 45128 Essen (DE)
(72) Erfinder: DYBALLA, Katrin, Marie, 45657 Recklinghausen (DE); FRANKE, Robert, 45772 Marl (DE); FRIDAG, Dirk, 45721 Haltern am See (DE); HESS,Dieter, 45770 Marl (DE); SELENT, Detlef, 18059 Rostock (DE); BÖRNER, Armin, 18059 Rostock (DE)

(57) **Zusammenfassung**

Verfahren zur Hydroformylierung von Cyclooctadien unter Einsatz des Liganden 4-([1,1':3',1"-Terphenyl]-2'-yloxy)-S-dinaphtho[2,1-d:1',2'-f][1,3,2]dioxaphosphepin (1).

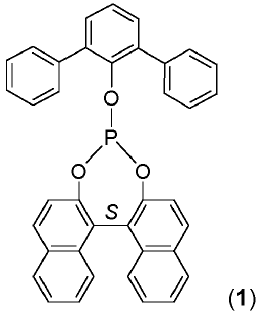

## Beschreibung

Die Erfindung betrifft ein Verfahren zur Hydroformylierung von Cyclooctadien (COD) unter Einsatz von 4-([1,1':3',1"-Terphenyl]-2'-yloxy)-S-dinaphtho[2,1-d:1',2'-f][1,3,2]dioxaphosphepin.

Phosphorhaltige Verbindungen spielen als Liganden in einer Vielzahl von Reaktionen eine entscheidende Rolle. Hierzu zählen Phosphitliganden, also Verbindungen, die P-O-Bindungen enthalten, die in der Hydrierung, Hydrocyanierung und vor allem in der Hydroformylierung Anwendung finden.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt. Als Katalysatoren in diesen Reaktionen werden häufig Verbindungen der Übergangsmetalle der VIII. Gruppe des Periodensystems der Elemente verwendet. Bekannte Liganden sind beispielsweise Verbindungen aus den Klassen der Phosphine, Phosphite und Phosphonite mit jeweils dreiwertigen Phosphor PIII. Eine gute Übersicht über den Stand der Hydroformylierung von Olefinen findet sich in R. Franke, D. Selent, A. Börner, "Applied Hydroformylation", Chem. Rev., 2012, DOI:10.1021/cr3001803.

Der Erfindung lag die Aufgabe zugrunde, ein Verfahren zur Hydroformylierung von Cyclooctadien bereitzustellen, welches einen guten Umsatz an Cyclooctadien liefert.

Die Aufgabe wird gelöst durch ein Verfahren nach Anspruch 1.

Verfahren zur Hydroformylierung von Cyclooctadien umfassend die Verfahrensschritte:
a) Vorlegen von Cyclooctadien;
b) Zugabe eines Komplexes umfassend:
   - ein Metallatom ausgewählt aus: Rh, Ru, Co, Ir, und
   - einen Liganden, welcher die Struktur (**1**) aufweist: 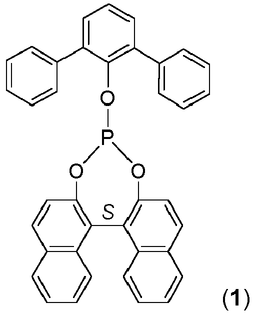 oder
      Zugabe einer Komplexvorstufe umfassend ein Metallatom ausgewählt aus: Rh, Ru, Co, Ir, und einer Verbindung welche die Struktur (**1**) aufweist: 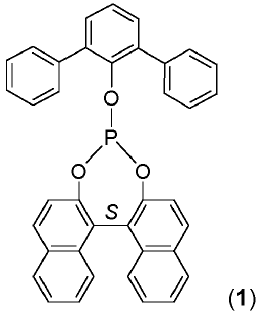
c) Zuführen von H₂ und CO,
d) Erwärmen des Reaktionsgemisches, wobei das Cyclooctadien zu einem Aldehyd umgesetzt wird.

Hierbei können die Verfahrensschritte a) bis d) in beliebiger Reihenfolge erfolgen.

In einer Variante des Verfahrens ist das Metallatom Rh.

In einer Variante des Verfahrens umfasst die Komplexvorstufe Cyclooctadien.

In einer Variante des Verfahrens handelt es sich bei der Komplexvorstufe um [(acac)Rh(COD)]. Hierbei stehen "acac" für Acetylacetonat-Anion und "COD" für Cyclooctadien.

In einer Variante des Verfahrens wird das Reaktionsgemisch im Verfahrensschritt d) auf eine Temperatur im Bereich von 50 °C bis 70 °C erwärmt.

Im Folgenden wird die Erfindung anhand von Ausführungsbeispielen näher erläutert.

### Analytik

### Chromatographie

Die präparativen flüssigkeitschromatographischen Trennungen via "Flashchromatographie" wurden mit einem Maximaldruck von 1.6 bar an Kieselgel 60 M (0.040-0.063 mm) der Firma Macherey-Nagel GmbH & Co, Düren durchgeführt. Die Trennungen ohne Druckbeaufschlagung wurden an Kieselgel Geduran Si 60 (0.063-0.200 mm) der Firma Merck KGaA, Darmstadt durchgeführt. Die als Eluentien verwendeten Lösungsmittel (Essigsäureethylester (technisch), Cyclohexan (technisch)) wurden zuvor destillativ am Rotationsverdampfer gereinigt.
Zur Dünnschichtchromatographie (DC) wurden PSC-Fertigplatten Kieselgel 60 F254 der Firma Merck KGaA, Darmstadt verwendet. Die Rf-Werte sind in Abhängigkeit vom verwendeten Laufmittelgemisch angegeben. Zur Anfärbung der DC-Platten wurde eine Cer-Molybdatophosphorsäure-Lösung als Tauchreagenz verwendet. Cer-Molybdatophosphorsäure-Reagenz: 5.6 g Molybdatophosphorsäure, 2.2 g Cer(IV)-sulfat-Tetrahydrat und 13.3g konzentrierte Schwefelsäure auf 200 mL Wasser.

### Gaschromatographie (GC/GCMS)

Die gaschromatographischen Untersuchungen (GC) von Produktgemischen und Reinsubstanzen erfolgte mit Hilfe des Gaschromatographen GC-2010 der Firma Shimadzu, Japan. Es wird an einer Quarzkapillarsäule HP-5 der Firma Agilent Technologies, USA (Länge: 30 m; Innendurchmesser: 0.25 mm; Filmdicke der kovalent gebundenen stationären Phase: 0.25 µm; Trägergas: Wasserstoff; Injektortemperatur: 250 °C; Detektortemperatur: 310 °C; Programm: Methode "hart": 50 °C Starttemperatur für 1 min, Heizrate: 15 °C/min, 290 °C Endtemperatur für 8 min) gemessen. Gaschromatographische Massenspektren (GCMS) von Produktgemischen und Reinsubstanzen wurden mit Hilfe des Gaschromatographen GC-2010 kombiniert mit dem Massendetektor GCMS-QP2010 der Firma Shimadzu, Japan aufgenommen. Es wird an einer Quarzkapillarsäule HP-1 der Firma Agilent Technologies, USA (Länge: 30 m; Innendurchmesser: 0.25 mm; Filmdicke der kovalent gebundenen stationären Phase: 0.25 µm; Trägergas: Wasserstoff; Injektortemperatur: 250 °C; Detektortemperatur: 310 °C; Programm: Methode "hart": 50 °C Starttemperatur für 1 min, Heizrate: 15 °C/min, 290 °C Endtemperatur für 8 min; GCMS: Temperatur der Ionenquelle: 200 °C) gemessen.

### Schmelzpunkte

Schmelzpunkte wurden mit Hilfe des Schmelzpunktbestimmungsgerätes SG 2000 der Firma HW5, Mainz gemessen und sind unkorrigiert.

### Elementaranalyse

Die Elementaranalysen wurden in der analytischen Abteilung des Institutes für Organische Chemie der Johannes Gutenberg-Universität Mainz an einem Vario EL Cube der Firma Foss-Heraeus, Haunau angefertigt.

### Massenspektrometrie

Alle Elektrosprayionisation-Messungen (ESI+) wurden an einem QTof Ultima 3 der Firma Waters Micromasses, Milford, Massachusetts durchgeführt. EI-Massenspektren sowie die hochaufgelösten EI-Spektren wurden an einem Gerät des Typs MAT 95 XL Sektorfeldgerät der Firma ThermoFinnigan, Bremen, gemessen.

### NMR-Spektroskopie

Die NMR-spektroskopischen Untersuchungen wurden an Multikernresonanzspektrometern des Typs AC 300 oder AV II 400 der Firma Bruker, Analytische Messtechnik, Karlsruhe, durchgeführt. Als Lösungsmittel wurde CDCl3 verwendet. Die 1 H- und 13C-Spektren wurden gemäß dem Restgehalt an nicht deuteriertem Lösungsmittel nach der NMR Solvent Data Chart der Fa. Cambridge Isotopes Laboratories, USA, kalibriert. Die Zuordnung der 1 H- und 13C-Signale erfolgte teilweise mit Hilfe von H,H-COSY, H,H-NOESY, H,C-HSQC und H,C-HMBC-Spektren. Die chemischen Verschiebungen sind als δ-Werte in ppm angegeben. Für die Multiplizitäten der NMR-Signale wurden folgende Abkürzungen verwendet: s (Singulett), bs (breites Singulett), d (Dublett), t (Triplett), q (Quartett), m (Multiplett), dd (Dublett von Dublett), dt (Dublett von Triplett), tq (Triplett von Quartett). Alle Kopplungskonstanten J wurden mit der Anzahl der eingeschlossenen Bindungen in Hertz (Hz) angegeben. Die bei der Signalzuordnung angegebene Nummerierung entspricht der in den Formelschemata angegebenen Bezifferung, die nicht mit der IUPAC-Nomenklatur übereinstimmen muss.

### Allgemeine Arbeitsvorschriften

Alle präparativen Arbeiten erfolgten unter Anwendung der Schlenk-Technik mit Argon als Schutzgas. Toluol und Tetrahydrofuran wurden mit einem Pure Solv MD-7 System gereinigt und bis zur Verwendung unter Argon aufbewahrt. Triethylamin wurde vor dem Einsatz unter Argon von Natriumketyl destilliert. Phosphortrichlorid (Aldrich) wurde vor dem Einsatz unter Argon destilliert. Alle präparativen Arbeiten erfolgten in ausgeheizten Gefäßen. Die Aufnahme von Kernresonanzspektren erfolgte an Bruker Avance 300 bzw. Bruker Avance 400, die gaschromatografische Analyse an Agilent GC 7890A, die Elementaranalyse an Leco TruSpec CHNS und Varian ICP-OES 715, und die ESI-TOF Massenspektrometrie an Thermo Electron Finnigan MAT 95-XP und Agilent 6890 N/5973 Geräten, die halbautomatische Säulenchromatografie an einem Teledyne Isco Combiflash Rf+.

### Synthesen

### a) (Anthracen-9-yloxy)dichlorophosphan (Vorstufe)

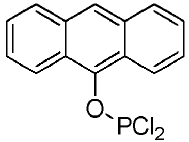

Zu einer gerührten Lösung von PCl₃ (5,16 g; 37,6 mmol) in THF (25 ml) wird bei 10 °C tropfenweise innerhalb von 90 min eine Mischung aus Anthron (2,03 g; 10,44 mmol) und Triethylamin (2 ml) in THF (80 ml) gegeben. Nach Stehenlassen über Nacht wird filtriert, das Filtrat im Vakuum zur Trockne gebracht und der erhaltene Rückstand in Toluol (50 ml) aufgenommen. Man filtriert erneut, entfernt das Lösungsmittel im Vakuum und trocknet den gelben Rückstand bei 50°C/ 0,1 mbar. Anschließend wird der erhaltene Feststoff mit Hexan (30 ml) bei Raumtemperatur über Nacht verrührt. Man filtriert und wäscht den Filterkuchen nochmals mit Hexan (3x20 ml) und trocknet. Ausbeute: 2,27 g (73%). ³¹P-NMR (CD₂Cl₂): 202,5 (s) ppm.

### b) 4-([1,1':3',1"-Terphenyl]-2'-yloxy)-S-dinaphtho[2,1-d:1',2'-f][1,3,2]dioxaphosphepin (Ligand 1)

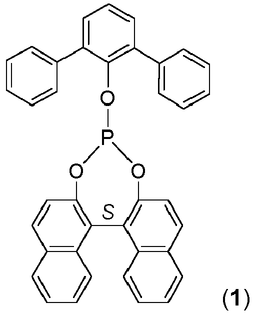

Zu einer Lösung von 2,6-Diphenylphenol (0,568 g; 2,30 mmol) in THF (7 ml) wird bei -20 °C unter Rühren eine 0,32 M Lösung von *n*-Butyllithium in Heptan (7,2 ml; 2,30 mmol) gegeben. Man rührt 20 min, lässt auf Raumtemperatur kommen und tropft die erhaltene Lösung unter Rühren zu einer auf-20°C gekühlten Lösung von 4-Chlor-*S*-dinaphtho[2,1-d:1',2'-*f*][1,3,2]dioxaphosphepin (0,849 g; 2,42 mmol) in THF (8 ml). Es wird 20 min bei -20°C und anschließend über Nacht bei Raumtemperatur gerührt. Man entfernt das Lösungsmittel im Vakuum, nimmt den Rückstand in Toluol (14 ml) auf, filtriert, engt zur Trockne ein und trocknet den erhaltenen Feststoff für 1 h bei 50°C/0,1 mbar. Die säulenchromatografische Aufarbeitung (Hexan/Toluol, Gradient Hexan 100 → 0 %; R*_{f}*= 0,6 für das 1:2-Gemisch der Eluenten) ergibt nach Entfernen der Lösungsmittel und Trocknen für 3 h bei 50°C/0,1 mbar 1,066 g (1,90 mmol; 82%). Elementaranalyse (ber. für C₃₈H₂₅O₃P= 560,586 g/mol): C 81,30 (81,42); H 4,71 (4,49); P 5,56 (5,53)%. ESI-TOF/HRMS: *m*/*e* 561,16060 (*M*+H)⁺.
³¹P-NMR (CD₂Cl₂): 146,4 (s) ppm.
¹H-NMR (CD₂Cl₂): 5,97 (d, *J*_{HH}= 8,8 Hz, 1 H); 6,86 (d, *J*_{HH}= 8,8 Hz, 1 H); 7,22-7,32 (m, 4 H); 7,34-7,39 (m, 1 H); 7,42-7,50 (m, 4H); 7,54-7,64 (m, 10H); 7,71 (d, *J*_{HH}= 8,8 Hz, 1H); 7,88-7,98 (m, 3H) ppm.
¹³C-NMR (CD₂Cl₂): 121,9; 122,2; 122,4; 124,6; 125,1; 125,2; 125,5; 126,4; 126,6; 127,0; 127,1; 127,); 128,6; 128,7; 128,9; 129,2; 129,4; 130,1; 130,6; 131,0; 131,2; 131,4; 131,9; 132,5; 132,9; 136,4; 138,8; 146,4 (d, *J*_{CP}= 8,3 Hz); 147,0; 148,2 (d, *J*_{CP}= 4,8 Hz) ppm.

### c) 4-(Anthracen-9-yloxy)-S-dinaphtho[2,1-d:1',2'-f][1,3,2]dioxaphosphepin (Ligand 2)

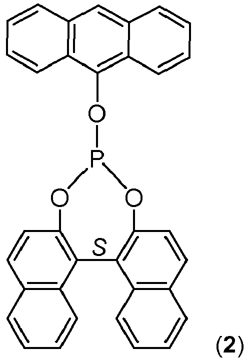

Eine bei -20 °C gerührte Suspension von Anthron (0,447 g; 2,30 mmol) in THF (5 ml) wird tropfenweise mit einer 0,32 M Lösung von *n*-Butyllithium in Heptan (7,2 ml; 2,30 mmol) versetzt. Man lässt auf Raumtemperatur kommen und tropft dann unter Rühren eine Lösung von 4-Chlor*S*-dinaphtho[2,1-*d*:1',2'-*f*][1,3,2]dioxaphosphepin (0,807 g; 2,30 mmol) in THF (6 ml) zu. Es wird über Nacht gerührt und filtriert. Man entfernt die flüchtigen Bestandteile des Filtrates im Vakuum, nimmt den erhaltenen gelben Feststoff in Toluol (10 ml) auf, filtriert über eine mit Kieselgel beschichtete G4-Fritte und engt das Filtrat im Vakuum ein. Der erhaltene Feststoff wird 3 h bei 50°C/0,1 mbar getrocknet. Die säulenchromatografische Aufarbeitung (Hexan/CH₂Cl₂, Gradient Hexan 100 → 0 %; R*_{f}* = 0,5 für das 1:1-Gemisch der Eluenten) ergibt 0,55 g (1,08 mmol; 47%) an reinem Produkt. Elementaranalyse (ber. für C₃₄H₂₁O₃P= 508,511 g/mol): C 80,80 (80,31); H 3,89 (4,16); P 6,02 (6,09)%. ESI-TOF/HRMS: m/e 509,12979 (*M*+H)⁺.
³¹P-NMR (CD₂Cl₂): 149,7 (s) ppm.
¹H-NMR (CD₂Cl₂): 7,37-7,42 (m, 2H); 7,51-7,65 (m, 9H); 7,87 (d, *J*_{HH}= 8,9 Hz, 1H); 8,02-8,14 (m, 6H); 8,40 (s, 1 H); 8,57 (d, *J*_{HH}= 8,9 Hz, 2H) ppm.
¹³C-NMR (CD₂Cl₂): 122,1; 122,4; 123,0; 123,6; 123,8; 124,7; 125,0; 125,7; 126,1; 126,5; 127,0; 127,3; 128,6; 128,9; 130,6; 131,1; 131,9; 132,3; 132,6; 133,1; 133,4; 143,5 (d, *J*_{CP}= 6,1 Hz); 147,4 (d, *J*_{CP}= 2,7 Hz); 148,3 (d, *J*_{CP}= 5,2 Hz) ppm.

### d) 2-([1,1':3,1"-Terphenyl]-2'-yloxy)-4,4,5,5-tetramethyl-1,3,2-dioxaphos-pholan (Ligand 3)

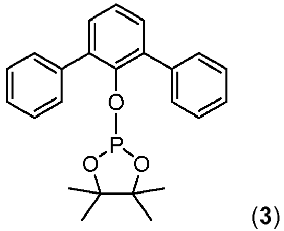

Das benötigte 2,6-Diphenylphen-I-oxydichlorphosphin wurde hergestellt wie beschrieben in W. Maringgele, A. Meller, Phosphorus, Sulfur and Silicon 1994, 90, 235-241.

Eine bei 0 °C gerührte Lösung von 2,6-Diphenylphen-l-oxydichlorphosphin (1,048 g; 3,018 mmol) in Toluol (18 ml) wird tropfenweise mit einer Mischung aus Pinacol (0,3405 g; 2,881 mmol) und Triethylamin (3,65 ml) in Toluol (12 ml) versetzt. Man lässt auf Raumtemperatur kommen, rührt über Nacht und filtriert. Das Filtrat wird eingeengt und der erhaltene Rückstand mehrfach aus heißem Heptan kristallisiert. Ausbeute: 0,295 g (0,752 mmol; 26%). Elementaranalyse (ber. für C₂₄H₂₅O₃P= 392,41 g/mol): C 73,31 (73,45); H 6,42 (6,42); P 7,53 (7,89) %. ESI-TOF/HRMS: m/e 393,16143, (M+H)⁺.
³¹P-NMR (CD₂Cl₂): 142,9 (s) ppm.
¹H-NMR (CD₂Cl₂): 1,17 (s, 6H), 1,31(s, 6H), 7,34-7,72 (m, 13H) ppm.
¹³C-NMR (CD₂Cl₂) δ 147,4 (d, *J*_{CP}= 6,5 Hz); 139,4; 136,6; 130,9; 130,8; 128,5; 127,6; 124,7; 85,3 (d, *J*_{CP}= 8,0 Hz); 25,5; 24,8 ppm.

### Hydroformylierung

Die Hydroformylierungsreaktionen wurden in einem mit Druckkonstanthaltung, Gasflussmessung, Begasungsrührer und Druckpipette ausgestatteten 200 ml-Autoklaven der Fa. Premex Reactor AG, Lengau, Schweiz, durchgeführt. Das als Lösungsmittel verwendete Toluol wurde mit einem Pure Solv MD-7 System gereinigt und unter Argon aufbewahrt.
Für die Versuche wurden im Autoklaven unter Argonatmosphäre Lösungen der Komplexvorstufe (= Katalysatorvorstufe) und des Liganden gemischt. Als Komplexvorstufe kam [(acac)Rh(COD)] (Umicore, acac = Acetylacetonat-Anion; COD= 1,5-Cyclooctadien) zum Einsatz. Für Versuche mit einer Konzentration von 100 ppm-m Rhodium wurden 5 ml einer 4,32 millimolaren Lösung in den Autoklaven gegeben. Anschließend wurde die einem Verhältnis L/Rh = 5:1 entsprechende Masse des Liganden in 20 ml Toluol gelöst und zugemischt. In eine druckfeste Pipette wurde eingefüllt: 2,69 g (24,86 mmol) COD-1,5. Der Autoklav wurde mit Synthesegas (Linde; H2 (Qualität 5.0) : CO (Qualität 4.7) = 1:1) auf einen Druck von 42 bar gebracht und auf 60 °C aufgeheizt. Nach Erreichen der Reaktionstemperatur wurde das Diolefin in den Autoklaven gepresst. Die Reaktion wurde bei konstantem Druck von 50 bar (Nachdruckregler der Fa. Bronkhorst, NL) ausgeführt. Der Autoklav wurde nach Ablauf der Reaktionszeit auf Zimmertemperatur abgekühlt, unter Rühren entspannt und mit Argon gespült. Jeweils 1 ml der Reaktionsmischungen wurden unmittelbar nach Abschalten des Rührers entnommen, mit 5 ml Pentan verdünnt und gaschromatographisch analysiert: HP 5890 Series II plus, PONA, 50 m x 0,2 mm x 0,5 µm.

Die Ergebnisse der Hydroformylierungsversuche sind in der nachfolgenden Tabelle zusammengestellt. Der angegeben Umsatz umfasst hierbei sowohl Monoaldehyde wie auch Dialdehyde.
Standard-Versuchsbedingungen: [Rh]= 0,717 x 10⁻⁴ M, Rh/Ligand/COD-1,5 = 1:5:1151, Lösungsmittel Toluol.

**Tabelle 1:**

| Ligand | p [bar] | T[°C] | t [h] | Umsatz COD-1,5 [%] |
|---|---|---|---|---|
| **1*** | 50 | 60 | 4 | 95 |
| **2** | 50 | 60 | 4 | 54 |
| **3** | 50 | 60 | 4 | 76 |

| | | | | |
|---|---|---|---|---|
| *) erfindungsgemäßes Verfahren | | | | |

Wie die Versuchsergebnisse zeigen, wird die Aufgabe durch das erfindungsgemäße Verfahren gelöst.

## Patentansprüche

1. Verfahren zur Hydroformylierung von Cyclooctadien umfassend die Verfahrensschritte:
a) Vorlegen von Cyclooctadien;
b) Zugabe eines Komplexes umfassend:
- ein Metallatom ausgewählt aus: Rh, Ru, Co, Ir, und
- einen Liganden, welcher die Struktur (**1**) aufweist: 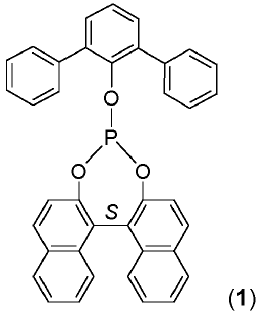 oder
Zugabe einer Komplexvorstufe umfassend ein Metallatom ausgewählt aus: Rh, Ru, Co, Ir, und einer Verbindung welche die Struktur (**1**) aufweist: 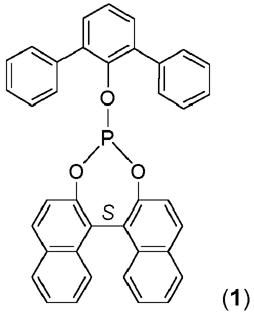
c) Zuführen von H₂ und CO,
d) Erwärmen des Reaktionsgemisches, wobei das Cyclooctadien zu einem Aldehyd umgesetzt wird.

2. Verfahren nach Anspruch 1,
wobei das Metallatom Rh ist.

3. Verfahren nach einem der Ansprüche 1 oder 2,
wobei die Komplexvorstufe Cyclooctadien umfasst.

4. Verfahren nach einem der Ansprüche 1 bis 3,
wobei es sich bei der Komplexvorstufe um [(acac)Rh(COD)] handelt.

5. Verfahren nach einem der Ansprüche 1 bis 4,
wobei das Reaktionsgemisch im Verfahrensschritt d) auf eine Temperatur im Bereich von 50 ° C bis 70 °C erwärmt wird.
